# EUROPÄISCHE PATENTANMELDUNG

(11) **EP 0 958 841 A2**
(43) Veröffentlichungstag der Anmeldung: **24.11.1999**
(21) Anmeldenummer: 99108650.5
(22) Anmeldetag: 14.05.1999
(51) Int. Cl.: A61M 16/06

(54) **Beatmungsmaske**

(30) Priorität: 18.05.1998 DE 19822308
(71) Anmelder: MAP Medizintechnik für Arzt und Patient GmbH & Co. KG, 82152 Martinsried (DE)
(72) Erfinder: Genger. Harald, D-82319 Starnberg (DE); Lang, Bernd C., D-82131 Gauting (DE)
(74) Vertreter: VOSSIUS & PARTNER

(57) **Zusammenfassung**

Die vorliegende Erfindung betrifft eine verbesserte Beatmungsmaske, die einen Maskengrundkörper, einen Beatmungsschlauch und ein Befestigungsmittel zur Befestigung der Maske am Kopf eines Patienten aufweist. Das Befestigungsmittel hat zwei miteinander über einen Verbindungsbereich verbundene Befestigungsbänder, wobei das erste an mindestens einem seiner Enden eine Öse aufweist, die an einem am Maskengrundkörper vorgesehenen Haken einhakbar ist, und das zweite den Maskengrundkörper an mindestens einer weiteren Stelle am Kopf des Patienten hält. Alternativ kann der Maskengrundkörper so ausgebildet sein, daß der Beatmungsschlauch an mindestens zwei beliebigen Anschlußstellen daran anschließbar ist, und die nicht benutzte Anschlußstelle mit einem Verschlußelement verschlossen ist.

## Beschreibung

Die vorliegende Erfindung betrifft eine Beatmungsmaske, die zur Beatmung eines Patienten über dessen Mund und/oder Nase anbringbar ist.

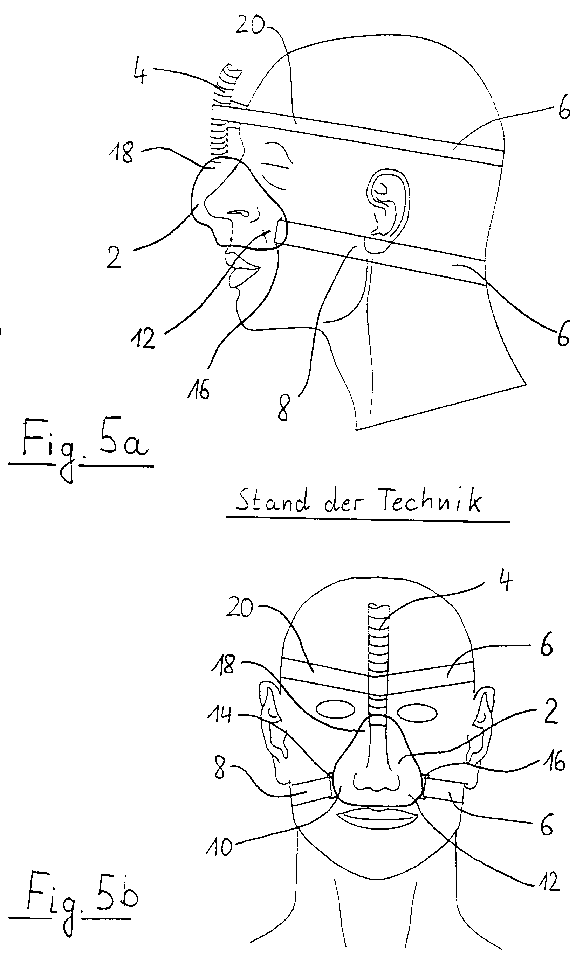

Herkömmliche Beatmungsmasken, wie z.B. in Fig. 5a und 5b dargestellt, bestehen im allgemeinen aus einem Maskenteil 2, der über die Nase und/oder den Mund eines zu beatmenden Patienten gebracht wird, einem Beatmungsschlauch 4 zur Bereitstellung von Beatmungsluft und/oder zum Abtransport von ausgeatmeter Luft, und Befestigungsmitteln 6 zum Befestigen der Beatmungsmaske 2 am Patienten. Der Maskenteil 2 ist durch einen entsprechenden Anpreßdruck durch die Befestigungsmittel 6 an die Gesichtsform eines Patienten im wesentlichen anpaßbar. Der Beatmungsschlauch 4 mündet, von oben kommend, in den Maskenteil 2. Die Befestigungsmittel 6 bestehen aus einem ersten elastischen Befestigungsband 8, das an den unteren beiden Ecken 10 und 12 des im wesentlichen dreieckigen Maskenteils 2 befestigt ist. Die Befestigung des Bandes 8 erfolgt am Maskenteil 2 durch zwei Ösen 14 und 16, die an den Ecken 10 und 12 des Maskenteils 2 vorgesehen sind. Zur Fixierung der oberen Ecke 18 des Maskenteils 2 ist ein zweites elastisches Befestigungsband 20 vorgesehen, das den Beatmungsschlauch 4 teilweise umschlingt. Die beiden Befestigungsbänder 8 und 20 werden jeweils um den Kopf des Patienten gespannt. Der Beatmungsschlauch 4 kann alternativ zur dargestellten Ausführungsform des Stands der Technik auch von unten an die Beatmungsmaske herangeführt werden.

Die Beatmungsmasken vom Stand der Technik, wie z.B. in Fig. 5a und 5b dargestellt, haben insbesondere den Nachteil, daß sie relativ schwierig am Kopf eines Patienten zu befestigen sind, weil die beiden Befestigungsbänder sich leicht miteinander verschlingen oder verdrehen, wenn die Maske über den Kopf des Patienten aufgesetzt wird. Dies kann insbesondere dann zu Problemen führen, wenn die Beatmungsmaske schnell am Patienten angebracht werden muß oder der Patient Kopfverletzungen hat. Ein weiterer Nachteil besteht darin, daß durch die Anordnung des oberen Befestigungsbandes der Beatmungsschlauch an die Stirn des Patienten gedrückt wird, so daß Druck- und Wundstellen entstehen können. Gleichfalls ist bei der Anordnung des Beatmungsschlauches nach unten die Gefahr der Druck- oder Wundstellen z.B. am Kinn des Patienten gegeben. Ferner ist nachteilig, daß die Befestigung der Beatmungsmaske mit dem Befestigungsband am Beatmungsschlauch nicht gewährleistet, daß der Maskenteil im wesentlichen an die Gesichtsform des Patienten angepaßt wird, sondern es besteht vielmehr die Möglichkeit, daß die obere Ecke des Maskenteils z.B. vom Gesicht des Patienten absteht.

Es ist daher die Aufgabe der vorliegenden Erfindung, eine verbesserte Beatmungsmaske zur Verfügung zu stellen, die insbesondere eine einfachere Handhabung, höhere Flexibilität, einen besseren Tragekomfort und eine erhöhte Funktionalität gewährleistet.

Zur Lösung dieser Aufgabe geht die Erfindung von dem Grundgedanken aus, daß die Beatmungsmaske einen Maskengrundkörper, einen Beatmungsschlauch und ein Befestigungsmittel zur Befestigung der Maske am Kopf eines Patienten aufweist. Das Befestigungsmittel weist zwei miteinander über einen Verbindungsbereich verbundene Befestigungsbänder auf. Das erste Befestigungsband hat an mindestens einem seiner Enden eine Öse, die in einem am Maskengrundkörper vorgesehenen Haken einhakbar ist. Das zweite Befestigungsband hält den Maskengrundkörper an mindestens einer weiteren Stelle, vorzugsweise am oberen Ende des Maskengrundkörpers mittels eines Bandführungselementes, am Kopf des Patienten.

Zur Lösung dieser Aufgabe geht die Erfindung ebenso von dem Grundgedanken aus, daß die Beatmungsmaske einen Maskengrundkörper, einen Beatmungsschlauch und ein Befestigungsmittel zur Befestigung der Maske am Kopf eines Patienten aufweist. Der Maskengrundkörper ist dabei so ausgebildet, daß der Beatmungsschlauch an mindestens zwei beliebigen Anschlußstellen daran anschließbar ist, wobei die nicht benutzte Anschlußstelle mit einem Verschlußelement verschlossen ist.

Diese beiden vorstehenden Lösungen werden unabhängig voneinander beansprucht; sie können jedoch auch im Rahmen der Erfindung miteinander kombiniert werden.

Vorteilhaft an der erfindungsgemäßen Beatmungsmaske ist insbesondere, daß die Maske einfach handhabbar ist, d.h. sie kann einfach am Kopf eines Patienten befestigt werden, einerseits indem die Befestigungsmittel einfach von oben über den Kopf des Patienten geschoben werden, und das untere Befestigungsband an den Haken eingehängt wird und andererseits, indem der Beatmungsschlauch entsprechend der äußeren Umstände in mehrere Richtungen vom Patienten weggeführt werden kann. Ein Verdrehen der Befestigungsbänder wird außerdem weitgehend unterbunden. Durch die Anordnung des oberen Befestigungsbandes direkt am Maskengrundkörper werden keine unangenehmen Druck- oder Wundstellen bewirkt und gleichzeitig ist die Maske besser an die Gesichtsform des Patienten angepaßt.

Die Erfindung wird im folgenden anhand einer bevorzugten Ausführungsform beispielhaft beschrieben. Die Zeichnungen zeigen in:
- Fig. 1: eine erfindungsgemäße Beatmungsmaske am Patienten mit von unten zugeführtem Beatmungsschlauch in Vorderansicht;
- Fig. 2: eine Seitenansicht von Fig. 1 mit von unten montiertem Beatmungsschlauch;
- Fig. 3: eine erfindungsgemäße Beatmungsmaske am Patienten mit von oben zugeführtem Beatmungsschlauch in Vorderansicht;
- Fig. 4: eine Seitenansicht der Beatmungsmaske von Fig. 3; und
- Fig. 5a und 5b: eine Beatmungsmaske vom Stand der Technik am Patienten.

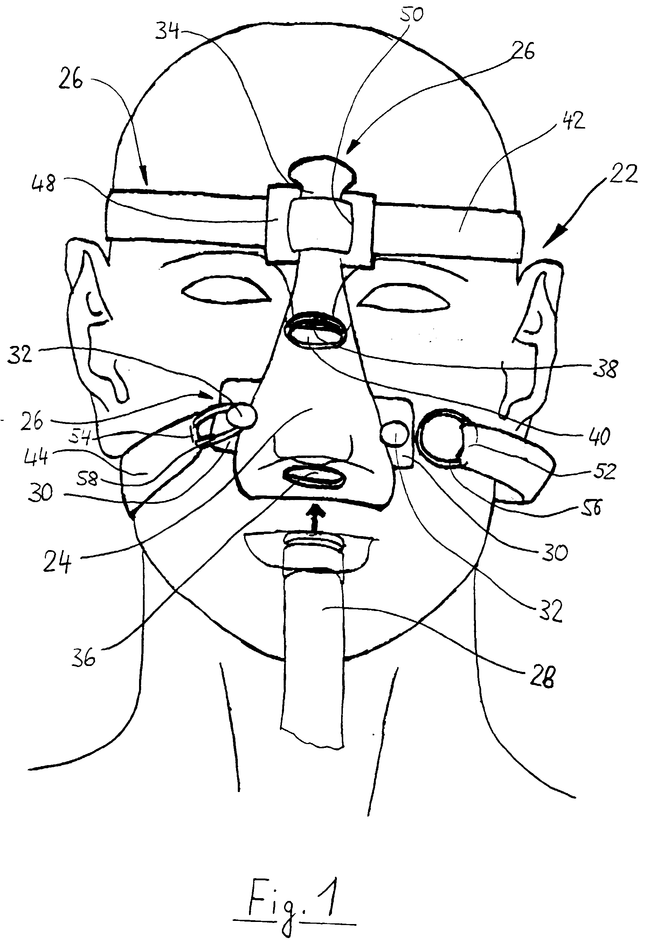

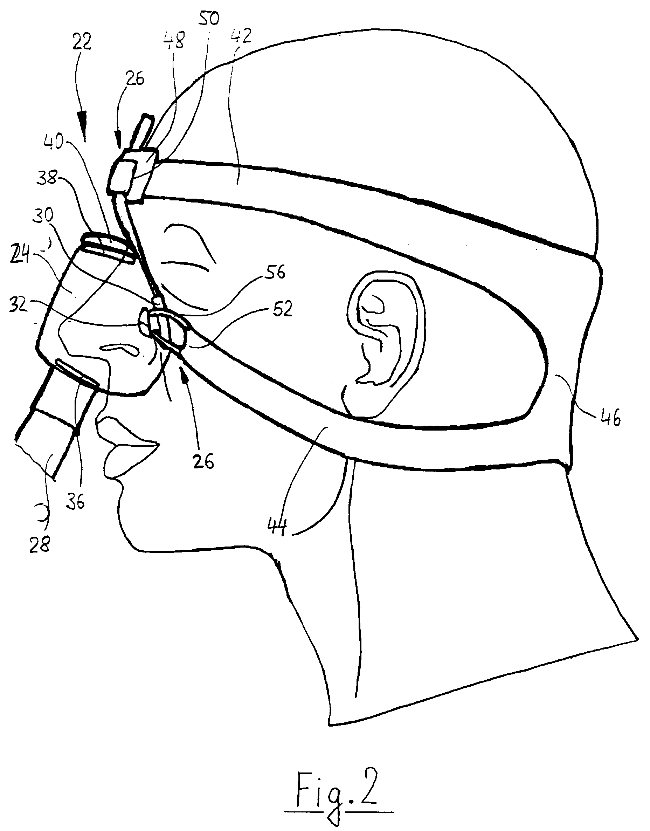

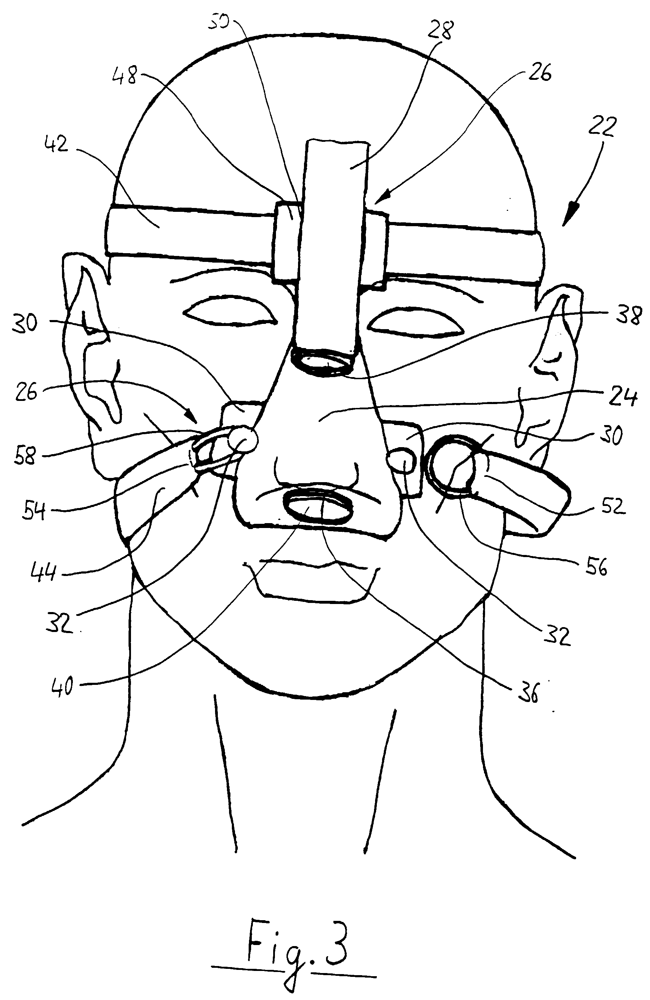

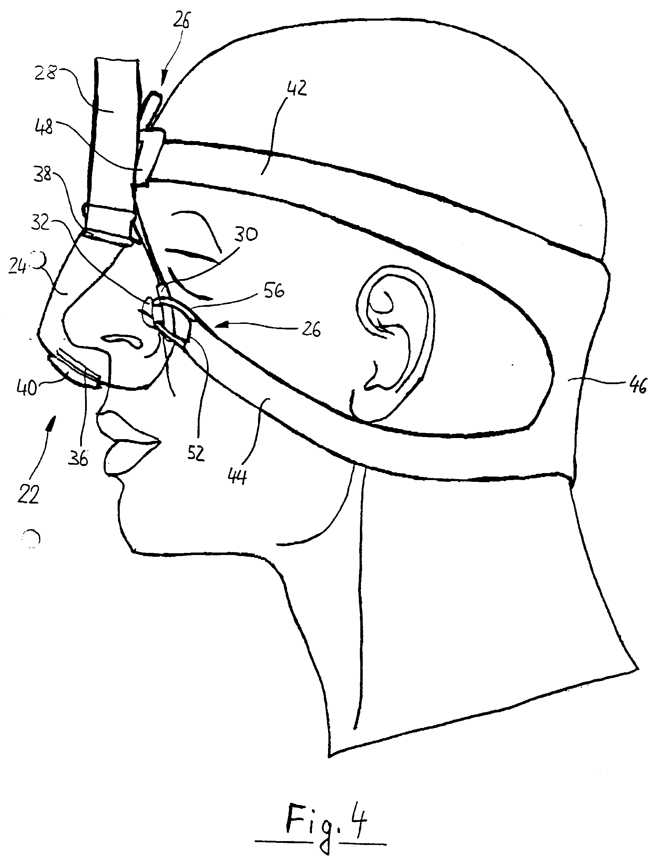

Die in den Figuren 1 bis 4 dargestellte erfindungsgemäße Beatmungsmaske 22 besteht im wesentlichen aus einem Maskengrundkörper 24, einem Befestigungsmittel 26 und einem Beatmungsschlauch 28. Der Maskengrundkörper 24 ist so ausgebildet, daß er über die Nase und/oder den Mund eines zu beatmenden Patienten gebracht werden kann und hat vorzugsweise eine im wesentlichen dreieckige Form. Im unteren Bereich des Maskengrundkörpers 24 sind beidseitige Vorsprünge 30 vorgesehen, an denen jeweils ein Haken 32 ausgebildet ist. Der Maskengrundkörper 24 weist im oberen Bereich einen etwas eingeschnürten Abschnitt 34 auf. Der Beatmungsschlauch 28 ist am Maskengrundkörper 24 an mindestens zwei beliebigen Anschlußstellen 36 und 38, vorzugsweise von unten oder oben, anschließbar. Die nicht benutzten Anschlußstelle, z.B. die obere Anschlußstelle in Figuren 1 und 2 oder die untere Anschlußstelle 36 in Figuren 3 und 4, wird mit einem Verschlußelement 40 verschlossen.

Durch die Anordnung von mehreren Anschlußstellen 36 und 38 am Maskengrundkörper 24 wird gewährleistet, daß der Beatmungsschlauch 28 entsprechend der äußeren Gegebenheiten bzw. den Erfordernissen des Patienten von beliebigen Seiten zugeführt werden kann. Somit wird ermöglicht, daß der Patient die Beatmungsmaske aufbehalten kann, selbst wenn z.B. bei einem Transport, im OP oder aus sonstigem Anlaß der Beatmungsschlauch aus einer anderen Richtung zugeführt werden muß.

Das Befestigungsmittel 26 zum Befestigen der Maske am Kopf des Patienten weist im wesentlichen ein oberes 42 und ein unteres Befestigungsband 44 auf, die durch einen Verbindungsbereich 46 miteinander verbunden sind. Der Verbindungsbereich 46 befindet sich etwa auf halber Länge der beiden Befestigungsbänder 42 und 44, d.h. wenn ein Patient die Beatmungsmaske 22 trägt, befindet sich der Verbindungsbereich 46 im Nacken oder Hinterkopfbereich des Patienten. Das obere Befestigungsband 42 ist vorzugsweise ein durchgehendes, elastisches Band, das in seinem vorderen Bereich ein Bandführungselement 48 zur Befestigung des Maskengrundkörpers 24 aufweist. Das Bandführungselement 48 hat zwei Schlitze 50, durch die das obere Befestigungsband 42 geführt ist. Indem das obere Band 42 zwischen den beiden schlitzen 50 aus dem Bandführungselement 48 herausgezogen wird, kann der verjüngte Abschnitt 34 des Maskengrundkörpers 24 durch die so entstandene Öffnung hindurchgeführt werden. Beim Aufsetzen der Maske wird das obere Befestigungsband 42 gespannt und der verjüngte Abschnitt 34 des Maskengrundkörpers 24 wird im Bandführungselement festgeklemmt, so daß die Beatmungsmaske 22 oben fixiert ist.

In einer bevorzugten Ausführungsform weist das untere Befestigungsband 44 zwei Enden 52 und 54 auf, an denen jeweils eine vorzugsweise aus elastischem Material geformte Öse 56 bzw. 58 angebracht ist. Zur unteren Lagefixierung der Beatmungsmaske 22 werden die Ösen 56 und 58 des unteren Befestigungsbandes 44 an den Haken 32 des Maskengrundkörpers 24 eingehängt.

Es ist jedoch auch möglich, das untere Befestigungsband 44 nur mit einem seiner Enden 52 oder 54 mit dem Maskengrundkörper 24 verbindbar auszugestalten, während das jeweilige andere Ende fest mit dem Maskengrundkörper 24 verbunden ist.

Es wird somit auf einfache Weise eine einfach anlegbare Beatmungsmaske 22 geschaffen, die gut an die Gesichtsform des Patienten anformbar ist. Durch die erfindungsgemäße Ausbildung des Befestigungsmittels 26 wird ferner ein schnelles Anlegen der Beatmungsmaske 22 ermöglicht.

Die Anordnung der Anschlußstellen unten 36 bzw. oben 38 wird sichergestellt, daß dem mit Beatmungsluft zu versorgenden Patienten unabhängig von den äußeren Gegebenheiten, der Beatmungsschlauch 28 aus verschiedenen Richtungen zugeführt werden kann.

Neben den zuvor beschriebenen elastischen Befestigungsbändern 42 und 44 können auch Befestigungsbänder, die in ihrer Länge einstellbar sind, verwendet werden. Ebenso ist es denkbar, anstelle des Bandführungselements 48 mit den Schlitzen 50, ein Haken-Ösen-System (Klettverschluß) oder sonstiges Befestigungsmittel vorzusehen. Auch für das Befestigen des unteren Bandes 44 sind im Rahmen der Erfindung andere schnell zu verbindende Befestigungssysteme geeignet. Beispielhaft werden hier Haken-Ösen-Systeme (Klettverschluß), Knöpf- oder Klemmverbindungen genannt.

## Patentansprüche

1. Beatmungsmaske (22) mit einem Maskengrundkörper (24), einem Beatmungsschlauch (28) und einem Befestigungsmittel (26) zur Befestigung der Maske am Kopf eines Patienten, wobei das Befestigungsmittel (26) zwei miteinander über einen Verbindungsbereich (46) verbundene Befestigungsbänder (42, 44) aufweist, das erste Befestigungsband (44) an mindestens einem seiner Enden (52 oder 54) eine Öse (56 oder 58) aufweist, die in einem am Maskengrundkörper (24) vorgesehenen Haken (32) einhakbar ist, und das zweite Befestigungsband (42) den Maskengrundkörper (24) an mindestens einer weiteren Stelle am Kopf des Patienten hält.

2. Beatmungsmaske (22) nach Anspruch 1, wobei der Verbindungsbereich (46) der beiden Befestigungsbänder (42, 44) auf halber Länge der Bänder (42, 44) vorgesehen ist.

3. Beatmungsmaske (22) nach Anspruch 1 oder 2, wobei die Befestigungsbänder (42, 44) elastisch sind.

4. Beatmungsmaske (22) nach einem der Ansprüche 1 bis 3, wobei die Öse (56 oder 58) aus elastischem Material vorgesehen ist.

5. Beatmungsvorrichtung (22) nach einem der Ansprüche 1 bis 4, wobei das erste Befestigungsband (44) an beiden Enden (52, 54) jeweils eine Öse (56, 58) und der Maskengrundkörper (24) beidseitig Haken (32) zum Einhaken des ersten Befestigungsbandes (44) aufweist.

6. Beatmungsmaske (22) nach einem der Ansprüche 1 bis 5, wobei das zweite Befestigungsband (42) durch ein Bandführungselement (48) geführt ist, in das ein eingeschnürter Abschnitt (34) des Maskengrundkörpers (24) hineinragt, so daß dieser durch die Spannung des Bandes (42) darin fixiert ist.

7. Beatmungsmaske (22) mit einem Maskengrundkörper (24), einen Beatmungsschlauch (28) und einem Befestigungsmittel (26) zur Befestigung der Maske am Kopf eines Patienten, insbesondere nach einem der Ansprüche 1 bis 6, wobei der Maskengrundkörper (24) so ausgebildet ist, daß der Beatmungsschlauch (28) an mindestens zwei beliebigen Anschlußstellen (36, 38) daran anschließbar ist und die nicht benutzte Anschlußstelle (36, 38) mit einem Verschlußelement (40) verschlossen ist.

8. Beatmungsmaske (22) nach Anspruch 7, wobei zwei Anschlußstellen (36, 38) vorgesehen sind.

9. Beatmungsmaske (22) nach Anspruch 7 oder 8, wobei eine erste Anschlußstelle (38) nach oben und eine zweite Anschlußstelle (36) nach unten gerichtet ist.

10. Beatmungsmaske (22) nach einem der Ansprüche 7 bis 9, wobei das Befestigungsmittel (26) zwei miteinander über einen Verbindungsbereich (46) verbundene Befestigungsbänder (42, 44) aufweist.
